# EUROPÄISCHE PATENTSCHRIFT

(11) **EP 0 902 817 B1**
(45) Veröffentlichungstag und Bekanntmachung des Hinweises auf die Patenterteilung: **23.05.2001**
(21) Anmeldenummer: 97925020.6
(22) Anmeldetag: 30.05.1997
(51) Int. Cl.: C09K 3/18

(54) **ENTEISUNGSMITTEL AUF DER BASIS VON ACETATEN UND/ODER FORMIATEN UND VERFAHREN ZUM SCHMELZEN VON SCHNEE UND EIS AUF VERKEHRSFLÄCHEN MIT HILFE DIESES MITTELS**
DE-/ANTIICING AGENT BASED ON ACETATES AND/OR FORMATES, AND PROCESS FOR MELTING, USING SAID AGENT, SNOW AND ICE ON AREAS FOR TRAFFIC
DEGIVRANT A BASE D'ACETATES ET/OU DE FORMIATES ET PROCEDE POUR FAIRE FONDRE DE LA NEIGE ET DU VERGLAS SUR DES ZONES DE CIRCULATION A L'AIDE DUDIT AGENT

(30) Priorität: 07.06.1996 DE 19622857; 25.11.1996 DE 19648716
(43) Veröffentlichungstag der Anmeldung: 24.03.1999
(73) Patentinhaber: Clariant GmbH, 65929 Frankfurt am Main (DE)
(72) Erfinder: STANKOWIAK, Achim, D-84503 Altötting (DE); BECKER, Wilfried, D-84524 Neuötting (DE)
(86) Internationale Anmeldenummer: EP9702810
(87) Internationale Veröffentlichungsnummer: WO9747703

(56) Entgegenhaltungen:
- EP-A- 0 375 214
- EP-A- 0 494 506
- WO-A-94/17152
- DE-A- 4 034 217
- US-A- 4 803 007
- US-A- 5 127 954
- US-A- 5 435 930

## Beschreibung

Die Erfindung betrifft ein Enteisungsmittel auf der Basis von Alkalimetallacetaten, Alkalimetallformiaten oder einer Mischung der beiden für Verkehrsflächen. Die Erfindung betrifft ferner ein Verfahren zum Schmelzen von Schnee und Eis auf Verkehrsflächen unter Verwendung dieses Mittels.

Schnee und/oder Eis auf Straßen, Radfahrwegen, Gehwegen, Brücken, Sportplätzen, Flugplätzen und dergleichen (im folgenden Verkehrsflächen genannt) führen zu einer wesentlichen Beeinträchtigung des Verkehrsablaufes und der Verkehrssicherheit. Es ist deshalb schon seit langem bekannt, auf solchen Flächen ein Mittel zum Schmelzen oder Auftauen von Schnee und Eis aufzubringen.

Die Forderungen, die ein Enteisungsmittel erfüllen sollte, sind sehr vielseitig. Die Materialien, aus denen die in Rede stehenden Flächen aufgebaut sind, beispielsweise Beton, dürfen durch das Enteisungsmittel nicht beschädigt oder gar zersetzt werden. Wesentlich ist ferner, daß eine korrodierende Wirkung auch auf Metalle ausgeschlossen ist. Da das Mittel ins Abwasser gelangen kann, ist die biologische Abbaubarkeit eine weitere Forderung. Wesentlich ist ferner, daß eine sehr rasche Auftauung erreicht wird. Von der wirtschaftlichen Seite her ist es schließlich notwendig, daß eine nur geringe Menge an Enteisungsmittel erforderlich ist und daß es wenig kostet.

Im Stand der Technik sind zahlreiche Alkalimetall- und Erdalkalimetallsalze von anorganischen und organischen Säuren als Enteisungsmittel beschrieben, zum Beispiel Natriumchlorid, Calciumchlorid, Natriumformiat, Calciumformiat, Natriumacetat, Magnesiumacetat, Natriumlactat und dergleichen. Diese Enteisungsmittel, insbesondere die Acetate und Formiate, erfüllen zwar mehrere der oben erwähnten Forderungen, sie lassen aber bezüglich Korrosion zu wünschen übrig. So wirken sie vor allem auf Leichtmetalle wie Aluminium und Magnesium mehr oder weniger stark korrodierend, was im Hinblick auf Verkehrsmittel wie Autos, Motorräder, Flugzeuge und dergleichen besonders nachteilig ist.

Es sind bereits Versuche unternommen worden, die erwähnten Probleme mit Hilfe von Inhibitoren zu lösen. So wird in EP-A 0 375 214 ein flüssiges Enteisungsmittel beschrieben, das im wesentlichen aus 45 bis 60 Gew.-% von einem Alkalimetallacetat und/oder Alkalimetallformiat, 0,1 bis 0,4 Gew.-% von einem Alkalimetallphosphat, 0,2 bis 0,6 Gew.-% von einem Alkalimetallnitrit und Wasser als Rest auf 100 Gew.-% besteht, Gewichtsprozente bezogen auf das Gewicht des Enteisungsmittels. Die Inhibitorwirkung von Alkalimetallphosphat und Alkalimetallnitrit läßt aber noch zu wünschen übrig, insbesondere im Hinblick auf Magnesium; nachteilig ist ferner der Nitritgehalt.

In DE-A 40 34 217 wird ein flüssiges oder festes Enteisungsmittel auf der Basis von wasserlöslichen Alkalisalzen der Ameisensäure und/oder Essigsäure beschrieben, wobei das Korrosionsinhibitorsystem aus wasserlöslichen Polycarbonsäuren und wasserlöslichen Alkali- oder Ammoniumsilicaten und/oder Alkali- oder Ammoniumcarbonaten besteht. Dieses chlorid- und nitritfreie Enteisungsmittel soll Bauwerke aus Beton, Bitumen oder Gestein nicht angreifen und auch metallische Werkstoffe wie Eisen, Kupfer, Aluminium oder Zink nicht angreifen. Die antikorrosive Wirkung beruht auf der Kombination von einer wasserlöslichen Polycarbonsäure wie Bernsteinsäure, Citronensäure oder Weinsäure und einem wasserlöslichen Silicat und/oder Carbonat. Im Falle von Leichtmetallen läßt auch sie zu wünschen übrig.

Erwähnt sei noch US-A 4 803 007, worin ein Enteisungsmittel auf der Basis von Natriumchlorid beschrieben wird, wobei als Korrosionsinhibitor eine Mischung aus einem zweiwertigen Metallsalz und einem Alkalimetallpolyphosphat eingesetzt wird. Als divalente Metalle werden unter anderen Calcium, Magnesium und Barium und als Gegenionen Borate, Metasilicate und Sulfate genannt. Diese Inhibitorkombination wirkt vor allem einer Korrosion der Eisenmetalle entgegen, weniger aber der Korrosion von Leichtmetallen wie Magnesium.

Es wurde nun gefunden, daß eine Mischung aus Alkalimetallphosphaten und Alkalimetallsilicaten ein besonders wirksamer Leichtmetall-Korrosionsinhibitor ist für ein Enteisungsmittel auf der Basis von Alkalimetallsalzen der Ameisensäure und/oder Essigsäure.

Das erfindungsgemäße Enteisungsmittel besteht im wesentlichen aus
a) 87 bis 99,45 Gew.-%, vorzugsweise 92 bis 97,9 Gew.-%, von einem Alkalimetallacetat und/oder Alkalimetallformiat,
b) 0,5 bis 10 Gew.-%, vorzugsweise 2 bis 7 Gew.-%, von einem Alkalimetallsilicat und
c) 0,05 bis 3 Gew.-%, vorzugsweise 0,1 bis 1 Gew.-%, von einem Alkalimetallphosphat.

Die Komponente a) ist vorzugsweise ein Alkalimetallformiat. Von den wasserlöslichen Alkalimetallformiaten und ebenso -acetaten sind jene des Natriums und Kaliums bevorzugt. Die Komponente a) ist also vorzugsweise ein Natriumformiat und/oder Kaliumformiat.

Die Komponente b) ist ein wasserlösliches Alkalimetallsilicat, wobei das Alkalimetall wiederum vorzugsweise Natrium oder Kalium ist. Geeignete Vertreter sind die Orthosilicate (mono-, di-, tri- und tetrabasisch), Disilicate bis Tetrasilicate und/oder die wasserlöslichen Metasilicate, die bevorzugt sind.

Die Komponente c) ist ein wasserlösliches Alkalimetallsalz der Phosphorsäure. Sie kann ein mono-, di- oder tribasisches Alkalimetallphosphat sein, wobei das Alkalimetall vorzugsweise Natrium oder Kalium ist.

Die Herstellung des erfindungsgemäßen Enteisungsmittels erfolgt durch Zusammenmischen der flüssigen oder festen Komponenten. Das Enteisungsmittel kann also in fester Form, zum Beispiel als Pulver oder Granulat, oder als wäßrige Lösung eingesetzt werden. Die Menge der drei und gegebenenfalls weiteren zweckmäßigen Komponenten im wäßrigen Enteisungsmittel (Gesamtkonzentration) kann innerhalb weiter Grenzen variieren. Sie richtet sich vor allem nach der Löslichkeit der Komponenten im Wasser (es soll eine im wesentliche klare Lösung vorliegen). Mit einer konzentrierteren Lösung wird man eine geringere Menge benötigen, um Eis und/oder Schnee aufzutauen, als mit einer weniger konzentrierten. Die bevorzugte Konzentration liegt demnach bei 15 bis 70 Gew.-%, vorzugsweise 25 bis 60 Gew.-%, Gewichtsprozente bezogen auf das Gewicht der Lösung. Der pH-Wert des wäßrigen Enteisungsmittels liegt im allgemeinen bei 7 bis 10. Sofern der genannte pH-Wert nach dem Zusammenmischen der Komponenten nicht ohnehin vorliegt, wird man ihn durch Zugabe von vorzugsweise einem Alkalimetallhydroxid wie Natrium- oder Kaliumhydroxid auf den gewünschten Wert einstellen.

Das erfindungsgemäße Verfahren zum Schmelzen von Schnee und Eis auf Verkehrsflächen ist dadurch gekennzeichnet, daß eine wirksame Menge des beschriebenen Enteisungsmittels (in fester oder flüssiger Form) auf der zu behandelnden Verkehrsfläche aufgebracht wird, das heißt eine solche Menge, daß die angestrebte Beseitigung von Eis und/oder Schnee erreicht wird. Diese Menge hängt vor allem von der Außentemperatur und der vorhandenen Eis- und/oder Schneemenge ab und liegt im allgemeinen bei 10 bis 100 g pro m² eis- und/oder schneebedeckter Fläche. Das Aufbringen des flüssigen Enteisungsmittels kann zum Beispiel mit Hilfe der üblichen Sprühfahrzeuge durchgeführt werden.

Das erfindungsgemäße Enteisungsmittel besitzt eine Reihe von Vorteilen. So erfüllt es die eingangs genannten Forderungen und weist neben kurzer Auftauzeit auch eine unerwartet hohe Inhibierung der Metallkorrosion, insbesondere von Magnesium, auf. Dies resultiert offensichtlich aus der Kombination der genannten Phosphate und Silicate in einer jeweils bestimmten Menge und daraus, daß dieses Inhibitorsystem gerade im Falle von Alkalimetallacetaten und/oder -formiaten hochwirksam ist. Das erfindungsgemäße Enteisungsmittel ist chlorid- und nitritfrei und auch für Flugzeugverkehrsflächen wie Start- und Landebahnen, Abstellplätze, Busrouten und dergleichen besonders geeignet. Es wird vorzugsweise in fester Form (Pulver, Granulat, Körnchen und dergleichen) eingesetzt.

Die Erfindung wird nun anhand von erfindungsgemäßen Beispielen und Vergleichsbeispielen noch näher erläutert.

Die Enteisungsmittel der nachstehenden erfindungsgemäßen Beispiele 1 bis 5 und der Vergleichsbeispiele 1 bis 3 wurden durch Mischen der Komponenten hergestellt. Die angegebenen Prozentmengen der einzelnen Komponenten sind Gewichtsprozente.

| Beispiel 1 | |
|---|---|
| 97,8 % | Natriumformiat |
| 2,0 % | Natriummetasilicat |
| 0,2 % | Trikaliumphosphat |

| Beispiel 2 | |
|---|---|
| 92,0 % | Kaliumformiat |
| 7,0 % | Natriummetasilicat |
| 1,0 % | Trikaliumphosphat |

| Beispiel 3 | |
|---|---|
| 90,0 % | Natriumformiat |
| 8,0 % | Kaliummetasilicat |
| 2,0 % | Trikaliumphosphat |

| Beispiel 4 | |
|---|---|
| 96,5 % | Natriumformiat |
| 0,5 % | Natriummetasilicat |
| 3,0 % | Trinatriumphosphat |

| Beispiel 5 | |
|---|---|
| 87,0 % | Natriumformiat |
| 10,0 % | Natriummetasilicat |
| 3,0 % | Dikaliumhydrogenphosphat |

| Vergleichsbeispiel 1 | |
|---|---|
| 97,8 % | Natriumformiat |
| 2,0 % | Calciummetasilicat |
| 0,2 % | Trikaliumphosphat |

| Vergleichsbeispiel 2 | |
|---|---|
| 99,0 % | Natriumformiat |
| 0,5 % | Natriummetasilicat |
| 0,5 % | Citronensäure |

| Vergleichsbeispiel 3 | |
|---|---|
| 97,8 % | Natriumchlorid |
| 2,0 % | Calciummetasilicat |
| 0,2 % | Trikaliumphosphat |

Die Enteisungsmittel der Beispiele 1 bis 5 und der Vergleichsbeispiele 1 bis 3 wurden bezüglich Magnesiumkorrosion getestet. Der Test erfolgte nach ASTM F483 (ASTM = American Society for Testing and Materials). Dabei wird der gewogene Prüfkörper in das zu prüfende Enteisungsmittel, das auf einer Temperatur von 35 °C gehalten wird, 24 Stunden lang bei Normaldruck eingetaucht, worauf wiederum sein Gewicht bestimmt wird. Das Ergebnis des Korrosionstestes wird als Gewichtsdifferenz der beiden Gewichtsbestimmungen in Milligramm pro cm² Prüfkörper pro 24 Stunden angegeben. Der Test wurde mit Magnesium AMS 4375 (chromatiert) und mit 15gew.%igen wäßrigen Lösungen der angegebenen Enteisungsmittel durchgeführt. Die Ergebnisse sind in der nachstehenden Tabelle zusammengefaßt und zeigen, daß die erfindungsgemäßen Enteisungsmittel bezüglich Magnesiumkorrosion sehr gut inhibiert sind. Was die Auftauzeit der erfindungsgemäßen Enteisungsmittel betrifft, so erfüllen sie das geforderte schnelle Auftauen von Eis und Schnee. Die erfindungsgemäßen Enteisungsmittel besitzen also eine überraschend hohe Inhibierung bezüglich Magnesiumkorrosion und die geforderte kurze Auftauzeit.

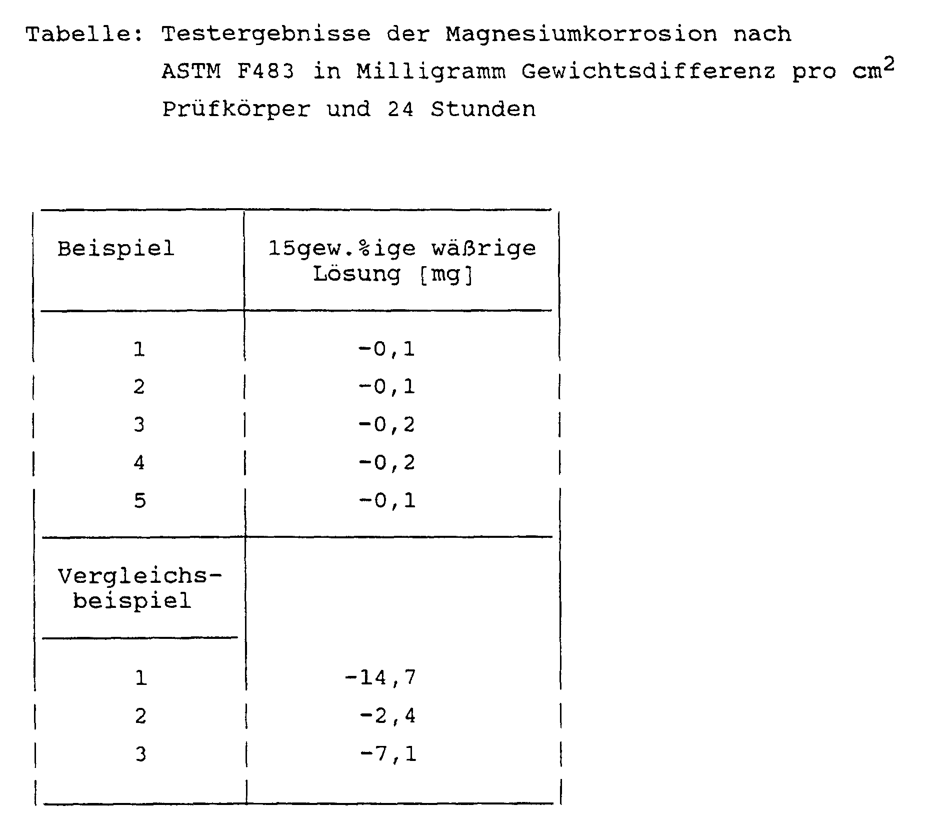

## Patentansprüche

1. Enteisungsmittel bestehend im wesentlichen aus
a) 87 bis 99,45 Gew.-% von einem Alkalimetallacetat oder einem Alkalimetallformiat oder einer Mischung davon,
b) 0,5 bis 10 Gew.-% von einem Alkalimetallsilicat und
c) 0,05 bis 3 Gew.-% von einem Alkalimetallphosphat.

2. Enteisungsmittel nach Anspruch 1, bestehend im wesentlichen aus
a) 92 bis 97,9 Gew.-% von einem Alkalimetallacetat oder einem Alkalimetallformiat oder einer Mischung davon,
b) 2 bis 7 Gew.-% von einem Alkalimetallsilicat und
c) 0,1 bis 1 Gew.-% von einem Alkalimetallphosphat.

3. Enteisungsmittel nach Anspruch 1 oder 2, dadurch gekennzeichnet, daß die Komponente a) ein Alkalimetallformiat, die Komponente b) ein Metasilicat und die Komponente c) ein mono-, di- oder tribasisches Alkalimetallphosphat ist.

4. Verfahren zum Schmelzen von Eis und Schnee auf Verkehrsflächen, dadurch gekennzeichnet, daß eine wirksame Menge von dem Enteisungsmittel nach Anspruch 1 in fester Form oder in Form einer wäßrigen Lösung auf die Verkehrsflächen aufgebracht wird.

## Claims

1. A deicing composition consisting essentially of
a) from 87 to 99.45% by weight of an alkali metal acetate or an alkali metal formate or a mixture thereof,
b) from 0.5 to 10% by weight of an alkali metal silicate, and
c) from 0.05 to 3% by weight of an alkali metal phosphate.

2. The deicing composition as claimed in claim 1, consisting essentially of
a) from 92 to 97.9% by weight of an alkali metal acetate or an alkali metal formate or a mixture thereof,
b) from 2 to 7% by weight of an alkali metal silicate, and
c) from 0.1 to 1% by weight of an alkali metal phosphate.

3. The deicing composition as claimed in claim 1 or 2, wherein component a) is an alkali metal formate, component b) is a metasilicate and component c) is a mono-, di- or tribasic alkali metal phosphate.

4. A method of melting ice and snow on traffic areas, which comprises applying an effective amount of the deicing composition as claimed in claim 1, in solid form or in the form of an aqueous solution, to the traffic areas.

## Revendications

1. Agent de dégivrage constitué essentiellement de
a) 87 à 99,45 % en poids d'un acétate de métal alcalin ou d'un formiate de métal alcalin ou d'un mélange de ceux-ci,
b) 0,5 à 10 % en poids d'un silicate de métal alcalin et
c) 0,05 à 3 % en poids d'un phosphate de métal alcalin.

2. Agent de dégivrage selon la revendication 1, constitué essentiellement de
a) 92 à 97,9 % en poids d'un acétate de métal alcalin ou d'un formiate de métal alcalin ou d'un mélange de ceux-ci,
b) 2 à 7 % en poids d'un silicate de métal alcalin et
c) 0,1 à 1 % en poids d'un phosphate de métal alcalin.

3. Agent de dégivrage selon la revendication 1 ou 2, caractérisé en ce que le composant a) est un formiate de métal alcalin, le composant b) est un métasilicate et le composant c) est un phosphate mono-, di- ou trialcalino-métallique.

4. Procédé pour faire fondre la glace ou la neige sur des zones de circulation, caractérisé en ce que l'on répand sur les zones de circulation une quantité efficace de l'agent de dégivrage selon la revendication 1 sous forme solide ou sous forme d'une solution aqueuse.
